# FASCICULE DE BREVET EUROPEEN

(11) **EP 0 663 238 B1**
(45) Date de publication et mention de la délivrance du brevet: **14.07.1999**
(21) Numéro de dépôt: 94403056.8
(22) Date de dépôt: 29.12.1994
(51) Int. Cl.: B01J 31/12, B01J 31/14

(54) **Procédé de préparation d'un catalyseur d'hydrogénation soluble en phase liquide organique**
Verfahren zur Herstellung eines in einer organischen flüssigen Phase löslichen Hydrierungskatalysators
Preparation process of a hydrogenation catalyst soluble in a liquid organic phase

(30) Priorité: 13.01.1994 FR 9400413
(43) Date de publication de la demande: 19.07.1995
(73) Titulaire: INSTITUT FRANCAIS DU PETROLE, 92502 Rueil-Malmaison (FR)
(72) Inventeur: Durand, Daniel, F-92500 Rueil Malmaison (FR); Hillion, Gérard, F-95220 Herblay (FR); Sarrazin, Patrick, F-92500 Rueil Malmaison (FR)
(74) Mandataire: Andreeff, François

(56) Documents cités:
- EP-A- 0 008 304
- EP-A- 0 368 419
- EP-A- 0 488 073
- EP-A- 0 531 174

## Description

La présente invention a pour objet un procédé de fabrication de catalyseurs d'hydrogénation en phase homogène, solubles en milieux organiques éventuellement saturés en eau dissoute dans le milieu réactionnel (eau discrète). Elle a également pour objet les catalyseurs obtenus par ce procédé, et le procédé d'hydrogénation.

Ces catalyseurs peuvent être mis en oeuvre, par exemple en hydrogénation sélective ou totale de composés insaturés mono ou polyoléfiniques, acétyléniques, aromatiques et de composés fonctionnels saturés ou insaturés tels que alcools, aldéhydes, acides organiques, cétones, phénols, esters, nitriles, les sulfones, les imines, les amines, les dérivés nitrés.

On connaît des catalyseurs solubles dans le milieu réactionnel qui sont préparés à partir de complexes de métaux précieux US-4,581,417 et US-4,631,315.

D'autres catalyseurs solubles peuvent être préparés à partir de métaux non précieux et en particulier à partir de métaux de transition tel que le fer, le cobalt et le nickel.

Pour que ces catalyseurs soient actifs en hydrogénation et solubles dans le milieu organique ils doivent être préparés par mise en contact d'un sel de ces métaux, et plus particulièrement d'un carboxylate, avec un composé réducteur de type alkylaluminium US-4,357,478 ou bien avec un composé organique de lithium ou de magnésium US-3,541,064.

Le brevet US-4,271,323 décrit également un procédé d'hydrogénation avec un catalyseur soluble dans l'heptane obtenu par réaction d'un composé réducteur organométallique de lithium, sodium et/ou aluminium réducteur, avec un mélange d'une part d'un composé (carboxylates généralement) de nickel, ou cobalt et d'autre part d'un composé de zinc, fer ou zirconium, manganèse ou molybdène.

Des composés réducteurs tels que l'hydrure de sodium ou l'hydrure mixte de sodium et d'aluminium peuvent être également mis en oeuvre US-4,258,226.

Pour hydrogéner sélectivement des copolymères de diènes conjugés et des alkyl aromatiques insaturés, des composés de nickel ou de cobalt peuvent être utilisés avec, comme agent réducteur, des alkylalumoxanes US-4,980,331 et US-5,030,779.

Tous ces composés présentent une activité, à des degrés divers, dans l'hydrogénation d'aromatiques ou d'hydrocarbures insaturés. Cependant en présence d'eau discrète (eau dissoute dans le milieu réactionnel), on observe progressivement une agglomération du catalyseur (non soluble) avec comme corollaire une diminution progressive de l'activité catalytique et, dans le cas de l'hydrogénation sélective de polyoléfines ou d'alcynes, une augmentation de la sélectivité de la réaction vers la formation de produits saturés indésirables.

On a maintenant découvert une nouvelle technique pour maintenir le catalyseur en parfaite condition de solubilité dans le milieu réactionnel. Cette technique consiste à neutraliser toutes les fonctions réductrices de l'agent réducteur qui n'auraient pas réagi avec les sels des métaux réductibles. Cette neutralisation est généralement opérée par introduction d'un agent oxydant après formation de l'espèce catalytique active. Elle a donc lieu en général avant le stockage du catalyseur ou avant son introduction dans le milieu réactionnel.

L'invention, en améliorant la solubilité du catalyseur en présence d'eau, permet d'accroître l'activité et la sélectivité du catalyseur. Elle permet, d'une part, d'éviter la formation de dépôts inopportuns de catalyseur rendu pratiquement inactif par son agglomération sur les parois du réacteur et des échangeurs, et d'autre part, de travailler avec des concentrations plus faibles en métaux, ce qui est également favorable car l'élimination des métaux dans un hydrogénat liquide homogène est toujours complexe.

L'invention a donc pour objet un procédé de préparation d'un catalyseur utilisable notamment pour l'hydrogénation en phase homogène, ledit catatyseur étant soluble en milieu organique comprenant :
- une étape (a) de mise en solution, d'une part, d'au moins un composé d'au moins un métal A dans au moins un solvant organique S, et d'autre part, d'au moins un composé réducteur R d'au moins un métal B dans au moins un solvant organique S',
- une étape (b) de mise en contact du composé réducteur avec le composé de métal A,
- une étape (d) de neutralisation des fonctions réductrices résiduelles du composé réducteur R par au moins un agent oxydant O.

Le procédé peut comporter avantageusement l'ajout d'un agent sélectivant P dans la solution du composé de métal A obtenue dans l'étape (a) avant sa réduction par le composé réducteur R de métal B, et/ou de préférence, l'ajout a lieu dans une étape (c) dans le produit issu de l'étape (b).

Les étapes, (b) de réduction du métal (A), (c) de sélectivation du catalyseur par l'agent (P), et (d) de neutralisation par le composé (O) sont de préférence suivies d'une étape de mûrissement, à une température comprise entre -20°C et 200°C (où 150°C préféré) pendant un temps compris entre 0,1 mn et 100 h, de préférence 1 mn et 75 h et de manière privilégiée 10 mn et 50h.

Les mûrissements ont pour objectif d'obtenir des intermédiaires de préparation stabilisé avant de passer à l'étape suivante de préparation.

Les solutions, du composé de métal A et du composé réducteur R peuvent être réalisées avec le même solvant ou avec deux solvants différents. Cependant, pour des raisons de pureté de produits, il est préférable d'utiliser le même solvant et si possible que ce solvant puisse être soit le produit à hydrogéner soit le produit issu de l'hydrogénation.

Les solvants S et S' permettant la solubilisation des composés de métal A et les réducteurs de métal B sont les hydrocarbures saturés ou insaturés, les composes monoaromatiques (benzène, toluène, xylènes), les alkylbenzènes saturés ou insaturés, les polyaromatiques (naphtalène), les composés mono ou polycycliques saturés ou insaturés (cyclohexane, cyclohexène, décaline et tétraline), les oxydes, les éthers (tetrahydrofuranne) ou les éthers oxydes (éthers méthyliques du diéthylène glycol).

Ces solvants, devront être exempts de traces d'agents oxydants tels que l'eau, les alcools, l'oxygène, la présence de ces agents oxydants ayant comme inconvénients de détruire initialement une partie de la fonction réductrice de l'agent réducteur et de pénaliser son action vis à vis de la réduction du composé métallique A. Les solvants seront de préférence désaérés et saturés en gaz inerte ou réducteur (azote, argon, hydrogène).

Le métal A est choisi parmi au moins un des métaux des groupes Ib, IIb, Vb, VIb, VIIb et VIII, et de préférence Ib, VIb, VIIb et VIII de la classification périodique, et plus particulièrement le fer, le cobalt et le nickel, le cuivre, le chrome, le molybdène, le manganèse et le zinc.

Ces métaux peuvent être introduits sous forme d'halogénures, de sulfures, d'acétylacétonates mais de préférence sous forme de carboxylates d'acides organiques possédant de 2 à 25 atomes de carbone. Parmi ces derniers nous pouvons citer les acétates, les octoates, les décanoates, les naphténates, les stéarates, les palmitates, les oléates, les benzoates.

Les concentrations de ces composés de métal A dans le solvant S et avant leur mise en contact avec le composé réducteur R, sont comprises entre 0,01 et 10 moles par litre et de préférence entre 0,05 et 5 moles par litre.

Les composés réducteurs R de métal B sont des dérivés organométalliques d'au moins un métal B choisi dans le groupe formé par le lithium, le sodium, l'aluminium et plus particulièrement des dérivés mixtes de l'aluminium et du sodium et/ou du lithium. Ils possèdent, au moins, une liaison carbone-métal ou hydrogène métal. Chacune de ces liaisons correspond à une fonction réductrice.

Parmi ces composés réducteur R, nous pouvons citer le triéthylaluminium, le triisobutylaluminium, le chlorodiéthylaluminium, le diéthylterbutoxyaluminium, le diéthoxyéthylaluminium, l'hydrure de diisobutylaluminium, l'hydrure mixte de lithium et d'aluminium, l'hydrure mixte de sodium et d'aluminium, l'hydrure mixte de sodium et de bore, le butyllithium, l'éthylate de sodium et leur dérivés de substitution soit par un radical hydrocarboné soit par un groupe alcoxy. Dans ces dérivés de substitution le degré de substitution maximum est égal à la somme des liaisons carbone-métal et/ou hydrogène-métal, moins une qui assurera la réduction du métal A.

Les solutions de ces composés réducteurs doivent être, comme ci-dessus, préparées en absence de composés oxydants et en particulier en absence d'air, d'eau, d'alcools,... La concentration en composé réducteur R est comprise entre 0,01 et 10 moles par litre et de préférence entre 0,05 et 5 moles par litres.

La seconde étape (b) consiste a faire réduire partiellement ou totalement le composé de métal A par le composé réducteur R de métal B, dans un état d'oxydation pour lequel il est le plus actif pour la réaction considérée. La mise en contact de ces 2 réactifs, toujours en absence d'autres composés oxydants, est réalisée en atmosphère neutre (par exemple sous argon ou azote) ou réductrice (par exemple sous hydrogène), soit par introduction du composé métallique A sur le composé réducteur R, soit inversement, soit encore par introduction simultanée des 2 réactifs dans une ligne de mélange ou dans un réacteur de préparation exempt d'autres produits oxydants. Dans tous les cas il est souhaitable que le milieu soit agité pour favoriser une réduction homogène du composé de métal A par l'agent réducteur R.

Pour que la réduction du métal A conduise à l'espèce catalytique la plus active il est souhaitable que le rapport entre le nombre de fonctions réductrices (liaison carbone-métal et/ou métal-hydrogène du composé R) et la somme des degrés d'oxydation des métaux A (entre leur état d'oxydation dans le composé utilisé et le degré zéro) soit compris entre 0,1 et 20 et d'une manière préférée entre 0,5 et 10.

La température de mise en contact du composé de métal A et du réducteur de métal B sera comprise entre -20°C et +200°C et de préférence entre 0 et 150°C.

Cette étape de réduction du métal A est de préférence conduite en atmosphère neutre ou réductrice. Ce milieu réducteur peut être réalisé par introduction d'hydrogène sous des pressions partielles comprises entre 0 et 200 bar, de préférence entre 0,01 et 50 bar et d'une façon privilégiée entre 0,1 et 10 bar.

Une étape de mûrissement sous atmosphère neutre ou réductrice permettra d'ajuster les conditions opératoires pour que le taux de réduction du métal A par le réducteur soit optimum.

L'ajout d'agent sélectivant a pour objectif d'introduire dans la composition catalytique et plus particulièrement dans le produit issu de la réduction du composé de métal A par l'agent réducteur R de métal B, un ou plusieurs éléments P, métalliques ou organiques, permettant de conférer au catalyseur final plus de sélectivité pour l'hydrogénation sélective de composés de type alcynes, ou polyoléniques, ou polyfonctionnels, en produits monooéfiniques, ou monofonctionels,...

Parmi les éléments métalliques pouvant jouer ce rôle nous pouvons citer les métaux des groupes IA et IIA et IIB de la classification périodique ainsi que des éléments tels que l'yttrium, le titane, le zirconium, le gallium, le germanium, l'arsenic, l'étain et le plomb, le phosphore et l'antimoine.

Les composants organiques apportant un gain de sélectivité peuvent être choisis dans les familles des phosphines, des phosphites et des amines, les composés soufrés tels que le thiophène, le diméthyl sulfure et des composés azotés tels que la pyridine et la pipéridine.

Le rapport atomique ou molaire (selon la nature de l'agent P) entre L'agent sélectivant P et le métal A sera compris entre 0,001 et 10 et de préférence entre 0,01 et 5.

Comme pour l'étape de réduction, l'étape (c) de sélectivation sera conduite sous atmosphère de gaz neutre ou réducteur avantageusement aux mêmes pressions partielles, et être suivie d'une étape de mûrissement.

L'agent sélectivant P peut être également introduit dans la solution du composé de métal A avant l'étape (b) de réduction par le composé réducteur de métal B. Cette solution est généralement adoptée lorsque l'agent sélectivant est un composé métallique.

L'étape (d) de neutralisation des fonctions réductrices résiduelles de l'agent réducteur R, encore présentes après réduction du métal A et éventuellement réaction avec l'agent sélectivant P, consiste à introduire un agent oxydant O qui permette d'oxyder l'excédent de composé réducteur R tout en maintenant l'ensemble de la préparation catalytique, soluble dans un milieu organique même saturé en eau discrète.

Ces agents oxydants O sont préférentiellement choisis dans les familles des alcools primaires ou secondaires à longues chaînes aliphatiques (nombre d'atomes de carbone, égal ou supérieur à 6) comme l'hexanol, le décanol ou le dodécanol, les métylheptanols ou les éthylhexanols, les alcools tertiaires comme le terbutanol ou le 2-3 diméthylbutanol 2, linéaires ou ramifiés, Les polyols tels que le butanediol 1-4, le néopentylglycol, le triméthylolpropane, le pentaérythritol, le monopropylène glycol, l'éthylène glycol, et éventuellement les alcools cycliques comme le cyclohexanol ou portés par un groupement aromatique comme les phenyl-propanols.

Ces agents oxydants, de préférence exempts d'eau sont introduits dans le milieu de préparation du catalyseur, sous atmosphère de gaz neutre ou d'hydrogène, identique à celle décrite dans l'étape (b).

La quantité d'agent oxydant O mise en oeuvre est généralement supérieure à la quantité nécessaire à l'oxydation totale du composé réducteur R n'ayant pas réagit lors de l'étape (b) de réduction du composé de métal A.

On ajoute une quantité d'ajout oxydant O telle que le rapport entre le nombre de fonctions oxydantes du composé O et le nombre de fonctions réductrices contenues dans l'agent réducteur R n'ayant pas intervenu dans la réduction du composé de métal A (nombre calculé) est compris entre 1 et 20 et de préférence entre 1,5 et 10.

Cette neutralisation est réalisée sous atmosphère de gaz neutre ou réducteur, ou :
- la pression partielle en hydrogène est comprise entre 0 et 200 bar, de préférence entre 0,01 et 50 bar et d'une façon privilégiée entre 0,1 et 10 bar,
- la température comprise entre -20°C et 200°C et de préférence entre 0 et 150°C.

Comme après les étapes (b) et (c) une étape de mûrissement est conduite selon les conditions de température et de pressions partielles en hydrogène définies ci-dessus; le temps de mûrissement est compris entre 0,1 minute et 100 heures, de préférence entre 1 minute et 75 heures et d'une manière privilégiée entre 10 minutes et 50 heures.

L'étape (d) de neutralisation des fonctions réductrices résiduelles de l'agent réducteur R encore présentes dans la solution de catalyseur après réduction du métal A par au moins un agent oxydant O conduisent généralement à un produit soluble dans le milieu hydrocarboné même saturé en eau. Si cette étape (d) de la préparation du catalyseur n'est pas réalisée, l'eau présente dans la charge pourra avoir un double effet désactivant:
- l'excédent de réducteur sera détruit brutalement par l'eau avec généralement une formation de produits peu ou pas solubles dans le milieu; les particules (par exemple d'hydroxyde d'aluminium si le réducteur est du triéthylaluminium) seront susceptibles d'emprisonner une partie du catalyseur actif,
- et/ou la réaction de l'eau sur le métal plus ou moins réduit et coordiné avec le réducteur ( A-R' ou R' pourrait être par exemple un radical Al-Et si le réducteur était du triéthylaluminium), pour donner une espèce moins active.

La caractéristique la plus importante du procédé de l'invention réside dans la dernière étape de préparation qui consiste en l'élimination des fonctions réductrices de l'agent réducteur R par un agent oxydant O tout en conservant au catalyseur ainsi préparé une parfaite solubilité et une activité constante dans le milieu réactionnel humide. Sans cette neutralisation, les fonctions réductrices résiduelles sont progressivement oxydées par l'eau présente dans la charge à hydrogéner avec pour conséquence la précipitation de cette fraction de catalyseur hydrolysée rendue inactive et formation d'agglomérats qui piègent plus ou moins une partie du catalyseur actif, abaissant ainsi les performances catalytiques de L'ensemble.

Le milieu réactionnel (ou phase liquide) humide est défini comme renfermant une quantité d'eau égale ou inférieure à la quantité maximale qui peut être présente sous forme dissoute et parfaitement homogène dans ce milieu organique.

L'invention a également pour objet un procédé d'hydrogénation sélective ou totale, en phase liquide et en phase homogène, de composés insaturés mono ou polyoléfiniques, acétyléniques, aromatiques, de composés fonctionnels saturés ou insaturés, avec un catalyseur préparé ainsi que décrit précédemment.

De façon avantageuse, ce catalyseur ainsi préparé permet de procéder à l'hydrogénation avec une phase liquide renfermant des quantités d'eau égales ou inférieures à la quantité maximale qui peut être présente sous forme dissoute et parfaitement homogène dans ce milieu organique. En d'autres termes, le procédé d'hydrogénation peut être conduit en présence d'eau discrète dans le milieu réactionnel.
Le procédé d'hydrogénation est conduit à une température comprise entre 0 et 400°C, et avantageusement entre 20 et 300°C, et sous une pression partielle d'hydrogène de 0,01 à 20 MPa et de préférence de 0,1 à 5 MPa.

Les exemples suivants illustrent l'invention. Les exemples 2 à 6 décrivent la préparation de catalyseurs selon l'art antérieur. Ils sont testés à titre de comparaison dans l'exemple 12.

### EXEMPLES

### Exemple 1 (invention)

Dans un ballon en verre parfaitement sec, et purgé à l'argon, on met en solution 0,1 mole de triéthylaluminium dans 1l de benzène parfaitement sec et désaéré.

Dans un second ballon également sec on introduit 0,1 mole d'octoate de nickel puis on purge à l'argon. On ajoute ensuite 1l de benzène parfaitement sec et désaéré.

Après dissolution, on introduit dans un troisième ballon parfaitement sec et purgé à l'hydrogène, équipé d'une agitation par barreau magnétique et placé dans un bain d'eau maintenue à environ 25°C, 100 cm³ de la solution d'octoate de nickel. On injecte ensuite progressivement 80 cm³ de la solution de triéthylaluminium de telle manière que le temps d'injection soit d'au moins 10 minutes et que la température du milieu n'excède pas 50°C; le ballon étant maintenu sous un léger balayage d'hydrogène à pression atmosphérique.

Après introduction de la totalité du réducteur, on augmente progressivement la température du bain jusqu'à 50°C puis on laisse en agitation pendant une heure.

Cette étape de mûrissement terminée, on injecte toujours sous un faible balayage d'hydrogène à pression atmosphèrique et à cette température de 50°C, de l'alcool terbutylique sec à raison de 1 mmole par minute et pendant 8 minutes.

L'injection d'alcool terminée, on laisse le produit en agitation sous atmosphère d'hydrogène et à 60°C pendant 2 heures avant utilisation de ce catalyseur fini.

### Exemple 2 (comparaison)

On reproduit la préparation ci dessus à l'exception de l'étape de neutralisation de l'excédent de réducteur AlEt3 par le terbutanol.

### Exemple 3 (pour comparaison ex 4 brevet US 4,357,478)

A une solution de 0,1 mole de décanoate de nickel et de 0,02 mole d'acide décanoïque dans 17 cm³ de benzène, on ajoute une solution de triethylaluminium de telle manière que le rapport Ni/Al soit égal à 3.

### Exemple 4 (pour comparaison avec exemple 1 brevet US-4,980,331 et 5,030,779 )

Dans un ballon en verre on introduit à 25°C, 0,05 mole d'éthyl 2 hexanoate de nickel en solution dans du cyclohexane puis du méthylalumoxane de telle manière que le rapport Al/Ni soit égal à 3. Après 30 minutes d'agitation on injecte du triéthylaluminium avec un rapport Ni/Al égal à 1. Le catalyseur est utilisé 30 minutes après sa préparation.

### Exemple 5 (comparaison avec Ex. 2 brevets US-3,541,064)

En solution dans du toluène on mélange 0,06 moles de naphténate de nickel avec 0,24 moles de butyllithium. Le catalyseur est mis en oeuvre après 5 minutes d'agitation à 30°C.

### Exemple 6

On reproduit la préparation de l'exemple 3 à l'exception que dans le produit obtenu on injecte l'équivalent de 0,2 moles de cyclohexanol et qu'un mûrissement d'une durée de 2 heures soit conduit à 50°C

### Exemple 7

On reproduit la préparation de l'exemple 4 à l'exception que dans le produit obtenu après les 30 minutes de contact suite à l'injection du triéthylaluminium, on injecte 0,4 mole d'isobutanol et on laisse mûrir 8 heures à température ambiante.

### Exemple 8

On reproduit la préparation de l'exemple 5 à l'exception que dans le produit obtenu on injecte L'équivalent de 0,5 mole d'isopropanol à 20°C puis on augmente la température du mélange à 60°C pendant 1 heure.

### Exemple 9

On prépare un catalyseur selon la méthode décrite dans l'exemple 1 à la seule différence que après mélange de la solution de nickel et d'alkylaluminium, le solvant étant du cyclopentène et la période de mûrissement à 10°C, on introduit 0,2 mole de pyridine qu'on laisse en agitation à température ambiante avant injection de l'alcool de neutralisation toujours à 10°C.

### Exemple 10 (comparatif)

la préparation décrite à l'exemple 9 est reproduite à l'exception de la phase de neutralisation par l'alcool.

### Exemple 11

On prépare un catalyseur selon la méthode décrite dans l'exemple 1 à la seule différence que toute la préparation est réalisée sous argon avec comme solvant du cyclododécatriène et que le composé de métal A est du naphténate de cobalt. L'étape de neutralisation est réalisée avec du cyclododécanol à une température de 120°C avec un temps d'agitation de 4 heures. L'hydrogénation totale de 1 litre de cyclododécatriène en cyclododécane est totale après une heure de réaction sous 30 bars d'hydrogène et à 180°C.

### Exemple 12

Au moyen de 2 pompes on injecte sous pression, à température ambiante, dans une canalisation connectée à un réacteur d'hydrogénation de benzène, qui se comportera comme un réacteur de préparation de catalyseur, une solution 0,001 molaire d'octoate de nickel dans du cyclohexane et une solution 0,001 molaire de triethylaluminium également dans du cyclohexane, de telle manière que le rapport molaire Al(Et)₃ / octoate soit de 1,5. Sur cette même canalisation mais à 2 m de distance de l'endroit où est réalisé le mélange métal-réducteur, on injecte au moyen d'une troisième pompe de l'alcool terbutylique de telle manière que le rapport molaire alcool/Al(Et)₃ soit de 2.

Le mélange catalytique est véhiculé vers le réacteur d'hydrogénation distant de quelques mètres. Le réacteur, parfaitement agité, est alimenté en continu par du benzène renfermant environ 400 ppm d'eau dissoute et par de l'hydrogène sous une pression de 20 bar. Ce benzène hydrogéné à 200°C en cyclohexane est éliminé en continu en phase vapeur. La concentration en catalyseur injectée en continu dans le réacteur en même temps que la charge de benzène est équivalente à 20 ppm de nickel pour que le taux d'hydrogénation de l'hydrocarbure aromatique soit supérieur à 98%.

### Exemple 13

On reproduit, dans les mêmes conditions de température, de pression et de vitesse spatiale des réactifs, ainsi qu'avec la même charge de benzène renfermant les 400 ppm d'eau, l'essai ci-dessus mais en ne réalisant pas l'injection de l'alcool terbutylique qui permet la neutralisation de l'excèdent de réducteur triéthylaluminium. On constate que pour obtenir le même rendement de l'hydrogénation du benzène en cyclohexane il est impératif que la concentration en nickel dans la charge de benzène entrant dans le réacteur soit d'au moins 50 ppm.

### Exemple 14

Les catalyseurs des exemples 1 à 10 sont testés, en batch, dans des réactions d'hydrogénation sélective ou totale de composés hydrocarbonés insaturés, en présence ou non d'eau discrète (limite inférieure de saturation) ; Les résultats de tests sont rassemblés dans le tableau ci-dessous. Les conditions opératoires sont identiques pour un même réactif, que le milieu contienne de l'eau ou non.

Les conditions opératoires :

| | | |
|---|---|---|
| ex 1 et 2 | T = 200°C | P = 2 MPa |
| ex 3 et 6 | T = 200°C | P = 1,5 MPa |
| ex 4 et 7 | T = 200°C | P = 3 MPa |
| ex 5 et 8 | T = 180°C | P = 1 MPa |
| ex 9 et 10 | T = 25°C | P = 1,5 Mpa |

| Exemples | | Conc. Mtx | Réactif | H₂O | Conversion | Sélectivité |
|---|---|---|---|---|---|---|
| | | m.atome/l | | +avec -sans | (%) | (%) |
| | | | | | | |
| 1 | Inv. | 5 | benzène | 800 ppm | 98 | 99,8 |
| 2 | comp. | 5 | " | 800 ppm | 85 | 99,7 |
| | | | | | | (cyclohexane) |
| 3 | comp. | 7 | toluène | + | 95 | 99,4 |
| 6 | inv. | 7 | " | + | 83 | 99,8 |
| | | | | | | (m.cyclohexane) |
| 4 | comp. | 8 | naphtalène | 300 ppm | 70 | 95 (tétraline) |
| 7 | inv. | 8 | " | 300 ppm | 95 | 98 (tétraline) |
| | | | | | | |
| 5 | comp. | 12,0 | cyclododécatriène | + | 85 | 99,9(cyclo dodécane) |
| 8 | inv. | 8,5 | " | + | 92 | 99,9 |
| | | | | | | |
| 9 | inv. | 2,0 | cyclopendiène | - | 99,9 | 96,5(cyclopentène) |
| | | 2,0 | " | + | 99,6 | 98,5 |
| 10 | inv. | 3,0 | " | - | 99,9 | 96 |
| | | 4,0 | " | + | 99,7 | 95,5 |

Les résultats ci-dessus illustrent l'amélioration apportée par la neutralisation de l'excédent de réducteur dans les réactions d'hydrogénation de composés insaturés réalisées en présence d'eau discrète, aussi bien en activité qu'en sélectivité.

## Revendications

1. Procédé de préparation d'un catalyseur en phase homogène, soluble en milieu organique procédé caractérisé en ce qu'il comprend :
• une étape (a) de mise en solution, d'une part, d'au moins un composé d'au moins un métal A dans au moins un solvant organique S, et d'autre part, d'au moins un composé réducteur R d'au moins un métal B dans au moins un solvant organique S',
• une étape (b) de mise en contact du composé réducteur R, avec le composé de métal A,
• une étape (d) de neutralisation des fonctions réductrices résiduelles du composé réducteur R par au moins un agent oxydant O.

2. Procédé selon la revendication 1, caractérisé en ce que un agent sélectivant P est ajouté dans la solution du composé de métal de l'étape (a).

3. Procédé selon l'une des revendications 1 à 2, caractérisé en ce que dans une étape (c ), un agent sélectivant P est ajouté au produit issu de l'étape (b).

4. Procédé selon l'une des revendications 1 à 3, caractérisé en ce que les étapes (b), (c) et (d) sont réalisées entre -20 et 200°C.

5. Procédé selon l'une des revendications 1 à 4, caractérisé en ce que les étapes (b), (c) et (d) sont suivies par une étape de mûrissement se déroulant entre -20 et 200°C et pendant un temps compris entre 0,1 mm et 100 h.

6. Procédé selon l'une des revendications 1 à 5, caractérisé en ce que le métal A est choisi parmi au moins un des métaux des groupes Ib, IIb, V b, VIb, VIIb et VIII de la classification périodique.

7. Procédé selon l'une des revendications 1 à 6, caractérisé en ce que le métal A est choisi parmi au moins un des métaux des groupes Ib, VIb, VIIb et VIII.

8. Procédé selon l'une des revendications 1 à 7, caractérisé en ce que le métal A est choisi dans le groupe formé par le fer, le cobalt, le nickel, le cuivre, le chrome, le molybdène, le manganèse et le zinc.

9. Procédé selon l'une des revendications 1 à 8, caractérisé en ce que les composés de métal A sont choisis dans le groupe formé par les halogénures, les sulfures, les acétylacétonates et les carboxylates d'acides organiques possédant de 2 à 25 atomes de carbone.

10. Procédé selon l'une des revendications 1 à 9, caractérisé en ce que les composés de métal A sont choisis dans le groupe formé par les acétates, les octoates, les décanoates, les naphténates, les stéarates, les palmitates, les oléates, les benzoates.

11. Procédé selon l'une des revendications 1 à 10, caractérisé en ce que le composé réducteur R est un dérivé organométallique d'au moins un métal B choisi dans le groupe formé par le lithium, le sodium, l'aluminium.

12. Procédé selon l'une des revendications 1 à 11, caractérisé en ce que le composé réducteur R est choisi dans le groupe formé par le triétylaluminium, le triisobutylaluminium, le chlorodiéthylaluminium, le diéthylterbutoxyaluminium, le diéthoxyéthylaluminium, l'hydrure de diisobutylaluminium, l'hydrure mixte de lithium et d'aluminium, l'hydrure mixte de sodium et d'aluminium, l'hydrure mixte de sodium et de bore, le butyllithium, l'éthylate de sodium et leurs dérivés de substitution par un radical hydrocarboné ou alcoxy.

13. Procédé selon l'une des revendications 1 à 12, caractérisé en ce que les solvants S et S' sont choisis dans le groupe formé par les hydrocarbures saturés ou insaturés, les composés mono aromatiques, les composés polyaromatiques, les alkylbenzènes saturés ou insaturés, les composés monocycliques saturés ou insaturés, les composés polycycliques saturés ou insaturés, les oxydes, les éthers, les éthers oxydes.

14. Procédé selon l'une des revendications 1 à 13, caractérisé en ce que la concentration de la solution en composé de métal A est comprise entre 0,01 et 10 moles par litre.

15. Procédé selon l'une des revendications 1 à 14, caractérisé en ce que la concentration de la solution en composé de réducteur R est comprise entre 0,01 et 10 moles par litre.

16. Procédé selon l'une des revendications 1 à 15, caractérisé en ce que la réduction du composé de métal A par l'agent réducteur R dans l'étape (b) est réalisée avec un rapport, entre le nombre de fonctions réductrices de l'agent réducteur R et la somme des degrés d'oxydation des métaux A, compris entre 0,1 et 20.

17. Procédé selon l'une des revendications 1 à 16, caractérisé en ce que la réduction du composé de métal A par l'agent réducteur R dans l'étape (b) est réalisée avec un rapport entre le nombre de fonctions réductrices de l'agent réducteur R et le degré d'oxydation du métal A, compris entre 0,5 et 10.

18. Procédé selon l'une des revendications 1 à 17, caractérisé en ce que le composé sélectivant P est un composé d'au moins un métal choisi parmi les métaux des groupes IA, IIA et IIB de la classification périodique, l'yttrium, le titane, le zirconium, le gallium, le germanium, l'arsenic, l'étain et le plomb, le phosphore et l'antimoine.

19. Procédé selon l'une des revendications 1 à 18, caractérisé en ce que le composé sélectivant P est un composé organique choisi dans le groupe formé par les phosphines, les phosphites, les amines, les composés soufrés et les composés azotés.

20. Procédé selon l'une des revendications 1 à 19, caractérisé en ce que le rapport atomique ou molaire entre l'agent sélectivant P et le composé de métal A est compris entre 0,001 et 10.

21. Procédé selon les revendications 1 à 20, caractérisé en ce que l'agent oxydant O est choisi dans le groupe formé par les alcools primaires ou secondaires à longues chaînes aliphatiques avec un nombre d'atomes de carbone au moins égal à 6, les alcools tertiaires linéaires ou ramifiés, les alcools cycliques, les alcools portés par un groupement aromatique et les polyols.

22. Procédé selon l'une des revendications 1 à 21, caractérisé en ce que la quantité d'agent oxydant O ajoutée est telle que le rapport entre le nombre de fonctions oxydantes du composé O et le nombre de fonctions réductrices résiduelles est compris entre 1 et 20

23. Procédé selon l'une des revendications 1 à 22, caractérisé en ce que Les étapes b, c, d, ainsi que les étapes de mûrissement sont réalisées en atmosphère de gaz neutre ou de gaz réducteur.

24. Procédé selon la revendication 23, caractérisé en ce que le gaz réducteur est de l'hydrogène, et que la pression partielle en hydrogène est comprise entre 0 et 200 bar.

25. Procédé pour l'hydrogénation sélective ou totale, en phase liquide et en phase homogène, de composés insaturés mono ou polyoléfiniques, acétyléniques, aromatiques, de composés fonctionnels saturés ou insaturés, avec un catalyseur préparé selon l'une des revendications 1 à 24.

26. Procédé pour l'hydrogénation selon la revendication 25 dans lequel la phase liquide et homogène, renferme des quantités d'eau égales ou inférieures à la quantité maximale qui peut être présente sous forme dissoute et parfaitement homogène dans ce milieu organique.

## Claims

1. Method of preparation of a catalyst in a homogeneous phase, soluble in an organic medium, which method is characterised in that it comprises:
. a step (a) of placing in solution of on the one hand at least one compound of at least one metal A in at least one organic solvent S and on the other hand of at least one reducing compound R of at least one metal B in at least one organic solvent S',
. a step (b) of placing the reducing compound R in contact with the metal A compound,
. a step (d) of neutralising the residual reducing functions of the reducing compound R with at least one oxidising agent O.

2. Method according to claim 1, characterised in that a selectivity agent P is added to the solution of the metal compound of step (a).

3. Method according to one of claims 1 to 2, characterised in that in a step (c), a selectivity agent P is added to the product resulting from step (b).

4. Method according to one of claims 1 to 3, characterised in that steps (b), (c) and (d) are carried out at between -20 and 200°C.

5. Method according to one of claims 1 to 4, characterised in that the steps (b), (c) and (d) are followed by a ripening step taking place at between -20 and 200°C and for between 0.1 min. and 100 hrs.

6. Method according to one of claims 1 to 5, characterised in that the metal A is selected from among at least one of the metals of groups Ib, IIb, Vb, VIb, VIIb and VIII of the periodic classification.

7. Method according to one of claims 1 to 6, characterised in that the metal A is selected from among at least one of the metals of groups Ib, VIb, VIIb and VIII.

8. Method according to one of claims 1 to 7, characterised in that the metal A is selected from the group formed by iron, cobalt, nickel, copper chrome, molybdenum, manganese and zinc.

9. Method according to one of claims 1 to 8, characterised in that the metal A compounds are selected from the group formed by halides, sulphides, acetylacetonates and carboxylates of organic acids with 2 to 25 carbon atoms.

10. Method according to one of claims 1 to 9, characterised in that the metal A compounds are selected from the group formed by acetates, octoates, decanoates, naphthanates, stearates, palmitates, oleates and benzoates.

11. Method according to one of claims 1 to 10, characterised in that the reducing compound R is an organometallic derivative of at least one metal B selected from the group formed by lithium, sodium and aluminium.

12. Method according to one of claims 1 to 11, characterised in the reducing compound R is selected from the group formed by triethylaluminium, triisobutylaluminium, chlorodiethylaluminium, diethylterbutoxyaluminium, diethoxyethylaluminium, diisobutylaluminium hydride, mixed lithium and aluminum hydride mixed sodium and aluminum hydride, mixed sodium and boron hydride, butyllithium, sodium ethylate and their derivatives of substitution by a hydrocarbonic or alkoxy radical.

13. Method according to one of claims 1 to 12, characterised in that the solvents S and S' and selected from the group formed by saturated or unsaturated hydrocarbons, monoaromatic compounds, polyaromatic compounds, saturated or unsaturated alkylbenzenes, saturated or unsaturated monocyclic compounds, oxides, ethers and oxidised ethers.

14. Method according to one of claims 1 to 13, characterised in that the concentration of metal A in the solution is between 0.01 and 10 moles per litre.

15. Method according to one of claims 1 to 14, characterised in that the concentration of reducing compound R in the solution is between 0.01 and 10 moles per litre.

16. Method according to one of claims 1 to 15, characterised in that the reduction of the metal A compound by the reducing agent R in step (b) is carried out with a ratio between the number of reducing functions of the reducing agent R and the sum of degrees of oxidation of the metals A of between 0.1 and 20.

17. Method according to one of claims 1 to 16, characterised in that the reduction of the metal A compound by the reducing agent R in step (b) is carried out with a ratio between the number of reducing functions of the reducing agent R and the degree of oxidation of the metal A of between 0.5 and 10.

18. Method according to one of claims 1 to 17, characterised in that the selectivity compound P is a compound of at least one metal selected from among the metals of groups IA, IIA and IIB of the periodic classification, yttrium, titanium, zirconium, gallium, germanium, arsenic, tin and lead, phosphor and antimony.

19. Method according to one of claims 1 to 18, characterised in that the selectivity compound P is an organic compound selected from the group formed by phosphines, phosphites, amines, sulphurous compounds and nitrated compounds.

20. Method according to one of claims 1 to 19, characterised in the atomic or molar ratio between the selectivity agent P and the metal A compound is between 0.001 and 10.

21. Method according to claims 1 to 20, characterised in that the oxidising agent O is selected from the group formed by primary or secondary long-chain aliphatic alcohols with a number of carbon atoms at least equal to 6, linear or ramified tertiary alcohols, cyclic alcohols, alcohols carried by an aromatic group and polyols.

22. Method according to one of claims 1 to 21, characterised in that the quantity of oxidising agent O added is such that the ratio between the number of oxidising functions of the O compound and the number of residual reducing functions is between 1 and 20.

23. Method according to one of claims 1 to 22, characterised in that steps b, c, d as well as the ripening steps are carried out in a neutral gas or reducing gas atmosphere.

24. Method according to claim 23, characterised in that the reducing gas is hydrogen, and that the partial pressure of hydrogen is between 0 and 200 bar.

25. Method for selective or total hydrogenation in liquid phase and in homogeneous phase, of unsaturated mono-olefinic or polyolefinic, acetylinic, aromatic compounds, and of saturated or unsaturated functional compounds, with a catalyst prepared according to one of claims 1 to 24.

26. Method of hydrogenation according to claim 25 in which the liquid and homogeneous phases include quantities of water equal to or less than the maximum quantity which can be present in dissolved form and perfectly homogeneous in said organic medium.

## Patentansprüche

1. Verfahren zur Herstellung eines Katalysators in homogener in organischem Medium löslicher Phase, dadurch gekennzeichnet, daß das Verfahren umfasst:
- eine Stufe (a) zum In-Lösung-Bringen einerseits wenigstens einer Verbindung wenigstens eines Metalls A in wenigstens einem organischen Lösungsmittel S und andererseits wenigstens einer reduzierenden Verbindung R wenigstens eines Metalls B in wenigstens einem organischen Lösungsmittel S',
- eine Stufe (b) der Kontaktierung der reduzierenden Verbindung R mit der Metallverbindung A und
- eine Stufe (d) der Neutralisierung der reduzierenden restlichen Funktionen der reduzierenden Verbindung R durch wenigstens ein oxidierendes Mittel O.

2. Verfahren nach Anspruch 1, dadurch gekennzeichnet, daß ein selektivierendes Agens P in der Lösung der Metallverbindung der Stufe (a) zugesetzt wird.

3. Verfahren nach einem der Ansprüche 1 bis 2, dadurch gekennzeichnet, daß in einer Stufe (c) ein selektivierendes Agens P dem aus der Stufe (b) stammenden Produkt zugesetzt wird.

4. Verfahren nach einem der Ansprüche 1 bis 3, dadurch gekennzeichnet, daß die Stufen (b), (c) und (d) zwischen -20 und 200°C durchgeführt werden.

5. Verfahren nach einem der Ansprüche 1 bis 4, dadurch gekennzeichnet, daß die Stufen (b), (c) und (d) gefolgt werden von einer Stufe der Reifung, die zwischen -20 und 200°C und über einen Zeitraum zwischen 0,1 min und 100 h abläuft.

6. Verfahren nach einem der Ansprüche 1 bis 5, dadurch gekennzeichnet, daß das Metall A gewählt ist aus wenigstens einem der Metalle der Gruppen Ib, IIb, Vb, VIb, VIIb und VIII des Periodensystems der Elemente.

7. Verfahren nach einem der Ansprüche 1 bis 6, dadurch gekennzeichnet, daß das Metall A gewählt ist aus wenigstens einem der Metalle der Gruppen Ib, VIb, VIIb und VIII.

8. Verfahren nach einem der Ansprüche 1 bis 7, dadurch gekennzeichnet, daß das Metall A gewählt ist aus der Gruppe, die durch Eisen, Kobalt, Nickel, Kupfer, Chrom, Molybdän, Mangan und Zink gebildet ist.

9. Verfahren nach einem der Ansprüche 1 bis 8, dadurch gekennzeichnet, daß die Verbindungen des Metalls A gewählt sind aus der Gruppe, die gebildet ist durch die Halogenide, die Sulfide, die Acetylacetonate und die Carboxylate organischer Säuren, die 2 bis 25 Kohlenstoffatome besitzen.

10. Verfahren nach einem der Ansprüche 1 bis 9, dadurch gekennzeichnet, daß die Verbindungen des Metalls A gewählt sind aus der Gruppe, die gebildet ist durch die Acetate, die Octoate, die Decanoate, die Naphtenate, die Stearate, die Palmate, die Oleate und die Benzoate.

11. Verfahren nach einem der Ansprüche 1 bis 10, dadurch gekennzeichnet, daß die reduzierende Verbindung R ein organometallisches Derivat wenigstens eines Metalls B ist, das gewählt ist aus der durch Lithium, Natrium und Aluminium gebildeten Gruppe.

12. Verfahren nach einem der Ansprüche 1 bis 11, dadurch gekennzeichnet, daß die reduzierende Verbindung R gewählt ist aus der Gruppe, die gebildet ist durch Triethylaluminium, Triisobutylaluminium, Chlordiethylaluminium, Diethyl-tert-butoxyaluminium, Diethoxyethylaluminium, Isobutylaluminiumhydrid, gemischtes Hydrid des Lithiums und des Aluminiums, gemischtes Hydrid von Natrium und Aluminium, gemischtes Hydrid von Natrium und Bor, Butyllithium, Natriumethylat und deren Substitutionsderivate durch ein kohlenwasserstoffhaltiges Radikal oder Alcoxy.

13. Verfahren nach einem der Ansprüche 1 bis 12, dadurch gekennzeichnet, daß die Lösungsmittel S und S' gewählt sind aus der Gruppe, die gebildet ist durch die gesättigten oder ungesättigten Kohlenwasserstoffe, die monoaromatischen Verbindungen, die polyaromatischen Verbindungen, die gesättigten oder ungesättigten Alkylbenzene, die monozyklischen gesättigten oder ungesättigten Verbindungen, die polyzyklischen gesättigten oder ungesättigten Verbindungen, die Oxide, die Ether und Etheroxide.

14. Verfahren nach einem der Ansprüche 1 bis 13, dadurch gekennzeichnet, daß die Konzentration der Lösung an der Verbindung des Metalls A zwischen 0,01 und 10 Mol pro Liter beträgt.

15. Verfahren nach einem der Ansprüche 1 bis 14, dadurch gekennzeichnet, daß die Konzentration der Lösung an reduzierender Verbindung R zwischen 0,01 und 10 Mol pro Liter beträgt.

16. Verfahren nach einem der Ansprüche 1 bis 15, dadurch gekennzeichnet, daß die Reduktion der Verbindung des Metalls A durch das Reduktionsmittel R in der Stufe (b) realisiert wird bei einem Verhältnis, zwischen der Anzahl von reduzierenden Funktionen des Reduktionsmittels R und der Summe der Oxidationsgrade der Metalle A, zwischen 0,1 und 20.

17. Verfahren nach einem der Ansprüche 1 bis 16, dadurch gekennzeichnet, daß die Reduktion der Metallverbindung A durch das reduzierende Mittel R in der Stufe (b) mit einem Verhältnis zwischen der Anzahl reduzierender Funktionen des Reduktionsmittels R und dem Oxidationsgrad des Metalls A, zwischen 0,5 und 10 realisiert wird.

18. Verfahren nach einem der Ansprüche 1 bis 17, dadurch gekennzeichnet, daß die selektivierende Verbindung P eine Verbindung wenigstens eines Metalls, gewählt aus den Metallen der Gruppe IA, IIA und IIB des Periodensystems der Elemente, Yttrium, Titan, Zirkonium, Gallium, Germanium, Arsen, Zinn und Blei, Phosphor und Antimon, ist.

19. Verfahren nach einem der Ansprüche 1 bis 18, dadurch gekennzeichnet, daß die selektivierende Verbindung P eine organische Verbindung ist, die gewählt ist aus der durch die Phosphine, die Phosphite, die Amine, die schwefelhaltigen Verbindungen und die stickstoffhaltigen Verbindungen gebildeten Gruppe.

20. Verfahren nach einem der Ansprüche 1 bis 19, dadurch gekennzeichnet, daß das Atom- oder Molarverhältnis zwischen dem selektivierenden Agens P und der Verbindung des Metalls A zwischen 0,001 und 10 beträgt.

21. Verfahren nach einem der Ansprüche 1 bis 20, dadurch gekennzeichnet, daß das oxidierende Mittel A gewählt ist aus der Gruppe, die gebildet ist durch die primären oder sekundären Alkohole mit langen aliphatischen Ketten mit einer Anzahl von Kohlenstoffatomen von wenigstens gleich 6, die tertiären, linearen oder verzweigten Alkohole, die zyklischen Alkohole, die Alkohole, die als Träger eine aromatische Gruppe zum Träger haben sowie die Polyole.

22. Verfahren nach einem der Ansprüche 1 bis 21, dadurch gekennzeichnet, daß die Menge an zugesetztem oxidierenden Agens O derart ist, daß das Verhältnis zwischen der Anzahl oxidierender Funktionen der Verbindung A und der Anzahl reduzierender restlicher Funktionen zwischen 1 und 20 beträgt.

23. Verfahren nach einem der Ansprüche 1 bis 22, dadurch gekennzeichnet, daß die Stufen b, c, d sowie die Reifungsstufen in einer Atmosphäre neutralen Gases oder reduzierenden Gases durchgeführt werden.

24. Verfahren nach Anspruch 23, dadurch gekennzeichnet, daß das reduzierende Gas Wasserstoff ist und daß der Wasserstoffpartialdruck an Wasserstoff zwischen 0 und 200 bar beträgt.

25. Verfahren zur selektiven oder totalen Hydrierung in flüssiger Phase und in homogener Phase von ungesättigten mono- oder polyolefinischen, acetylenischen, aromatischen Verbindungen, von gesättigten funktionellen oder ungesättigten Verbindungen mit einem Katalysator, hergestellt nach einem der Ansprüche 1 bis 24.

26. Verfahren zur Hydrierung nach Anspruch 25, bei dem die flüssige und homogene Phase Mengen von Wasser umfaßt, die gleich oder geringer als die Maximalmenge ist, die in gelöster und vollständig homogener Form in diesem organischen Medium vorhanden sein kann.
